(19) Europäisches Patentamt
European Patent Office
Office européen des brevets

(11) EP 1 681 062 A1

(12) # EUROPEAN PATENT APPLICATION
published in accordance with Art. 158(3) EPC

(43) Date of publication:
**19.07.2006 Bulletin 2006/29**

(21) Application number: **04773347.2**

(22) Date of filing: **15.09.2004**

(51) Int Cl.:
***A61K 38/12*** (1995.01) ***A61P 31/04*** (2000.01)
***A61P 17/10*** (2000.01) ***A61P 27/02*** (2000.01)
***A61P 19/02*** (2000.01) ***A61P 35/00*** (2000.01)

(86) International application number:
**PCT/JP2004/013861**

(87) International publication number:
**WO 2005/025598 (24.03.2005 Gazette 2005/12)**

(84) Designated Contracting States:
**AT BE BG CH CY CZ DE DK EE ES FI FR GB GR HU IE IT LI LU MC NL PL PT RO SE SI SK TR**

(30) Priority: **16.09.2003 JP 2003323063**

(71) Applicants:
- **Astellas Pharma Inc.**
  **Tokyo 103-8411 (JP)**
- **SEKISUI CHEMICAL CO., LTD.**
  **Osaka-shi,**
  **Osaka 530-8565 (JP)**

(72) Inventors:
- **HATANO, Kazuo,**
  **Astellas Pharma Inc.**
  **Tokyo 103-8411 (JP)**
- **WATANABE, Yuji,**
  **Astellas Pharma Inc.**
  **Tokyo 103-8411 (JP)**
- **SAKUMA, Shozo,**
  **Astellas Pharma Inc.**
  **Tokyo 103-8411 (JP)**
- **YOKOTA, Y.,**
  **Astellas Pharma Inc.**
  **Tokyo 103-8411 (JP)**

(74) Representative: **Gille, Christian**
**Gille Hrabal Struck Neidlein Prop Roos**
**Bruckneistrasse 20**
**40593 Düsseldorf (DE)**

(54) **ANTIBACTERIAL DRUG FOR PROPIONIBACTERIUM ACNES**

(57) The present invention relates to an antibacterial agent for *Propionibacterium acnes* containing, as an active ingredient, a peptide represented by the formula [I]:

EP 1 681 062 A1

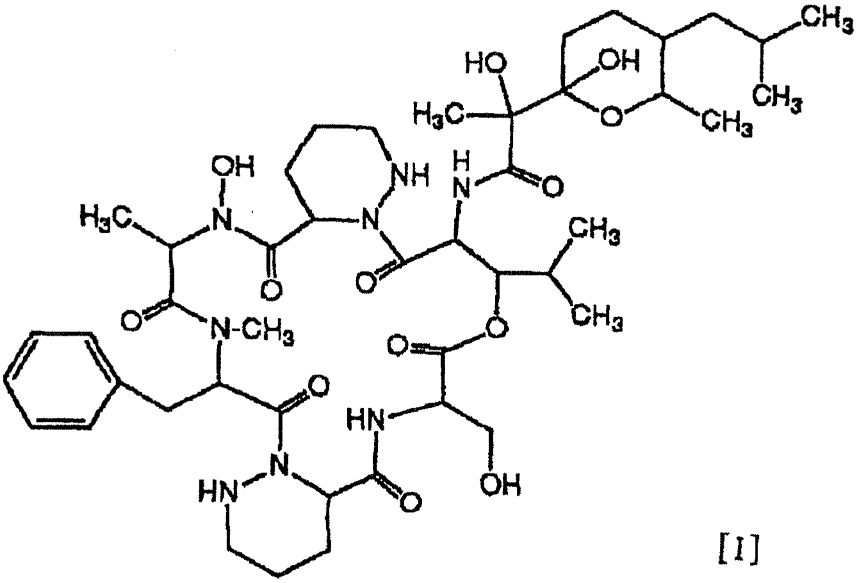

[I]

or a pharmaceutically acceptable salt thereof.

## Description

### Technical Field

The present invention relates to an antibacterial agent for *Propionibacterium acnes.* More specifically, the present invention relates to an antibacterial agent for *Propionibacterium acnes* comprising, as an active ingredient, a peptide represented by the formula [I] described below.

### Background Art

*Propionibacterium acnes* (*P. acnes;* acne bacillus), an anaerobic Gram-positive asporogenic bacillus, is indigenous to the skin and the gut. Although *P. acnes* had been considered to be essentially of low pathogenicity to humans, recent research has demonstrated that it plays an important role in the onset and progression of some chronic diseases. *P. acnes* potently activates the immune system, for example, 1) it activates complements, 2) it produces a neutrophil chemotactic factor and 3) it induces enzyme release from neutrophils. As examples of diseases mediated by *P. acnes* in the onset or progression thereof, acne, sarcoidosis, uveitis, conjunctivitis, keratitis, corneal phlycten, endocarditis, spondylarthritis and the like can be mentioned.

For this reason, there is a demand for the development of a drug that exhibits excellent antibacterial action on *P. acnes.*

In particular, acne is a chronic inflammatory disease that affects sebaceous hair follicles, with skin rashes often developing in the face, neck, and thoracodorsal portion. A wide variety of skin rashes such as comedos, red papules, pustules, indulation, nodules, cystomas, and scars coexist to various degrees. In usual cases of acne, closed comedos are formed from small comedos and progress to open comedos or to red papules and pustules, which are inflammatory comedos, and, in serious cases, sequelae such as acne scars persists. Additionally, because acne can be a cause of mental stress in the youth and others, appropriate treatment is required.

In acne, *P. acnes* in hair follicles produces lipase and decomposes triglycerides in sebum components to free fatty acids. The free fatty acids stimulate hair follicle infundibular epithelium to cause hyperkeratosis and induces comedo formation. Furthermore, *P. acnes* produces neutrophil chemotactic factors, complement activation factors, proteases, hyaluronidases and the like, resulting in the induction of neutrophils to lesion sites and the destruction of follicular epithelium. As a result, abscess is formed in the lesion sites (see Shigeo Ikeda, eds., "*Hyojun Hifukagaku* (6th edition)", pages 270 to 272, published by Igaku-Shoin, 2001).

Accordingly, as a therapeutic agent for acne, a drug that exhibits not only antibacterial action on *P. acnes,* but also hyperkeratosis suppressive action and suppressive action on inflammation due to neutrophil induction, is desired.

However, conventional therapeutic methods for acne, for example,

1) application of an external antibiotic preparation, an external synthetic antibacterial preparation, or a retinoid preparation,
2) oral administration of a tetracycline antibiotic agent or a macrolide antibacterial agent,
3) application of a sulfur and camphor lotion with defatting action,

and the like, are faulty because of the issue of adverse effects, limited efficacy, and ineffectiveness for severe acne. For this reason, some therapeutic methods may be used in combination, but this approach has drawbacks, including possibility of side effects due to drug interactions and expensiveness as a whole.

Sarcoidosis is a systemic disease characterized by non-caseous-necrotic epithelioid cell granuloma, with the lungs being the most commonly affected organ, but focuses involve the eyes, skin, lymph nodes, bones and joints, heart, nervous tissue, and other visceral organs. Uveitis occurs as part of the systemic lesions of sarcoidosis. Sarcoidosis is a type IV allergic disease in which T cells are activated by some causal factors, monocytes are accumulated locally by the cytokines produced by the activated T cells, followed by differentiation into macrophages and subsequent epithelioid cell granuloma formation. Although the cause of sarcoidosis has long been unknown, *P. acnes* has recently been drawing attention as the cause. It has been shown that *P. acnes* cells are collected from sarcoidosis lesion lymph nodes at high efficiency in large amounts, and that large amounts of *P. acnes* cell components are accumulated in epithelioid cell granuloma. Sarcoidosis is an intractable disease with no effective therapeutic method established. About 70% of sarcoidosis patients unavoidably undergo follow-up, with steroid hormones being used as the first-choice drug, which, however, are problematic with respect to side effects and the like (see *Atarashii Ganka,* Vol. 17 (extra issue), pages 87 to 89, 2000).

On the other hand, a peptide represented by the formula [I] below:

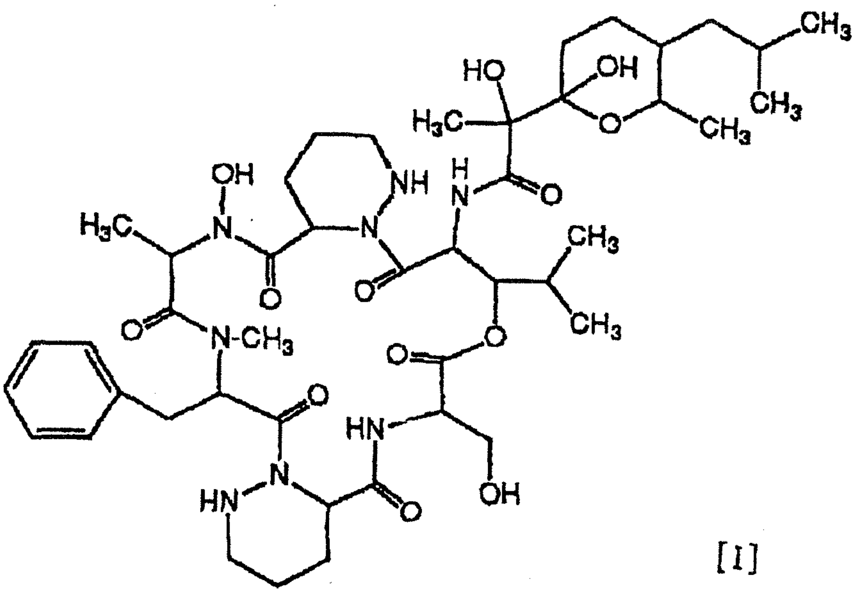

[I]

is disclosed in the WO96/12732 pamphlet and the like.

The WO96/12732 pamphlet discloses an antibiotic substance, antitumor substance, anti-inflammatory substance and wound-healing substance comprising a peptide represented by the formula [I] (hereinafter referred to as "peptide [I]"). Also disclosed are an antibacterial preparation, antitumor preparation, anti-inflammatory preparation, wound-healing preparation, and anti-ulcer preparation comprising peptide [I]. Furthermore, antibacterial effects of the peptide [I], particularly antibacterial effects on aerobic Gram-positive bacteria and allergic and anallergic inflammation inhibitory effects are disclosed.

JP-A-10-067677 discloses a treating agent for infectious skin disease comprising the peptide [I] and a therapeutic agent for infectious skin disease.

JP-A-10-120575 discloses a therapeutic agent for wounds comprising an oily and fatty base and refined sugar, which further comprises peptide [I].

JP-A-10-120590 discloses an external preparation for the treatment of parasitic skin disease comprising a therapeutic drug for parasitic skin disease and peptide [I].

JP-A-10-259141 discloses an external preparation for the treatment of skin disease comprising adrenocortical hormone, peptide [I], vitamin E and squalane.

In view of the above-described circumstances, it is an object of the present invention to provide an excellent antibacterial agent for *P. acnes.* It is another object of the present invention to provide a prophylactic or therapeutic agent for a disease caused by *P. acnes,* for example, acne, sarcoidosis and the like. It is still another object of the present invention to provide a prophylactic or therapeutic agent for acne that exhibits antibacterial action on *P. acnes,* hyperkeratosis suppressive action, and suppressive action on inflammation due to neutrophil induction.

## Disclosure of the Invention

With the aim of accomplishing the above-described objects, the present inventors conducted diligent investigations, and found that peptide [I] exhibits very excellent antibacterial action on *P. acnes,* and that peptide [I] exhibits suppressive action on epidermal keratinocyte growth and suppressive action on inflammation accompanied by neutrophil infiltration, confirmed that peptide [I] is particularly useful in the prevention or treatment of acne, and developed the present invention.

Accordingly, the present invention relates to the following:

(1) An antibacterial agent for *Propionibacterium acnes* comprising, as an active ingredient, a peptide represented by the formula [I]:

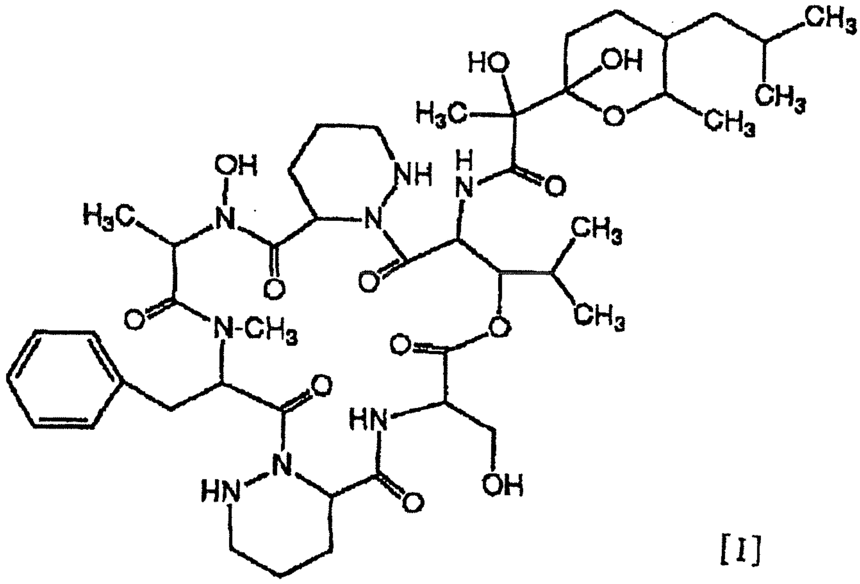

[I]

or a pharmaceutically acceptable salt thereof.

(2) A prophylactic or therapeutic agent for a disease resulting from *Propionibacterium acnes* infection, which comprises as an active ingredient a peptide represented by the formula [I] or pharmaceutically acceptable salt thereof.

(3) The prophylactic or therapeutic agent of (2) above, wherein the disease resulting from *Propionibacterium acnes* infection is a disease selected from the group consisting of acne, sarcoidosis, uveitis, conjunctivitis, keratitis, corneal phlycten, endocarditis and spondylarthritis.

(4) The prophylactic or therapeutic agent of (3) above, wherein the disease resulting from *Propionibacterium acnes* infection is acne.

(5) The prophylactic or therapeutic agent of (4) above, wherein the acne is acne vulgaris.

(6) The prophylactic or therapeutic agent of (3) above, wherein the disease resulting from *Propionibacterium acnes* infection is sarcoidosis.

(7) The prophylactic or therapeutic agent of (6) above, wherein the sarcoidosis is ocular sarcoidosis.

(8) The agent of any of (1) to (7) above, wherein the dosage form is an external preparation.

(9) A cosmetic for the prevention or treatment of acne comprising a peptide represented by formula [I] or a pharmaceutically acceptable salt thereof as an active ingredient.

(10) A prophylactic or therapeutic method for *Propionibacterium acnes* infection comprising using a peptide represented by formula [I] or a pharmaceutically acceptable salt thereof.

(11) A prophylactic or therapeutic method for a disease resulting from *Propionibacterium acnes* infection comprising using a peptide represented by formula [I] or a pharmaceutically acceptable salt thereof.

(12) A prophylactic or therapeutic method for acne comprising using a cosmetic comprising a peptide represented by formula [I] or a pharmaceutically acceptable salt thereof as an active ingredient.

(13) A use of a peptide represented by formula [I] or a pharmaceutically acceptable salt thereof for producing an antibacterial agent for *Propionibacterium acnes.*

(14) A use of a peptide represented by formula [I] or a pharmaceutically acceptable salt thereof for producing a prophylactic or therapeutic agent for a disease resulting from *Propionibacterium acnes* infection.

(15) A use of a peptide represented by formula [I] or a pharmaceutically acceptable salt thereof for producing a cosmetic for the prevention or treatment of acne.

(16) A commercial package comprising an antibacterial agent composition for *Propionibacterium acnes* comprising a peptide represented by formula [I] or a pharmaceutically acceptable salt thereof and a pharmaceutically acceptable carrier, and a document stating that the composition can be used or should be used for the prevention or treatment of *Propionibacterium acnes* infection.

(17) A commercial package comprising a pharmaceutical composition for the prevention or treatment of a disease resulting from *Propionibacterium acnes* infection comprising a peptide represented by formula [I] or a pharmaceutically acceptable salt thereof and a pharmaceutically acceptable carrier, and a document stating that the composition can be used or should be used for the prevention or treatment of a disease resulting from *Propionibacterium acnes* infection.

(18) A commercial package comprising a cosmetic composition for the prevention or treatment of acne comprising a peptide represented by formula [I] or a pharmaceutically acceptable salt thereof and a pharmaceutically acceptable carrier, and a document stating that the composition can be used or should be used for the prevention or treatment of acne.

According to the present invention, an excellent antibacterial agent for *P. acnes* can be obtained. Additionally, a prophylactic or therapeutic agent for a disease resulting from *P. acnes* infection, such as acne or sarcoidosis; a prophylactic or therapeutic agent for acne that exhibits antibacterial action on *P. acnes,* hyperkeratosis suppressive action, and suppressive action on inflammation due to neutrophil induction; and a cosmetic for the prevention or treatment of acne can be obtained.

**Detailed Description of the Invention**

The antibacterial agent for *P. acnes,* prophylactic or therapeutic agent for a disease resulting from *P. acnes* infection, and cosmetic for the prevention or treatment of acne of the present invention comprise peptide [I] as an active ingredient.

Peptide [I] can be obtained by culturing a peptide-[I]-producing strain belonging to the genus *Streptomyces,* for example, the actinomycete *Streptomyces nobilis* (hereinafter abbreviated as *"S. nobilis"*), subjecting an extract, which is extracted with an organic solvent from the obtained culture broth or a dried product of the broth or a cultured bacterium, to various column chromatographies, and recrystallizing a column chromatography fraction containing the desired product.

The actinomycete *S. nobilis* producing peptide [I] is available from public preservation organizations; for example, a preserved strain of the Institute of Physical and Chemical Research (JCM4274) (also preserved under ATCC19252 in the United States and under CBS198.65 in the Netherlands) and the like can be used.

Peptide [I] can be obtained by the methods described in the WO96/12732 pamphlet or Japanese Patent Unexamined Publication No. HEI-10-175996 and the like.

As the pharmaceutically acceptable salt of peptide [I], a commonly used salt that is non-toxic and pharmaceutically acceptable is suitable; for example, salts with alkali metals such as sodium or potassium; salts with alkaline earth metals such as calcium or magnesium; salts with inorganic bases such as ammonium; salts with organic amines such as triethylamine, pyridine, picoline, ethanolamine, triethanolamine, dicyclohexylamine, and N,N'-dibenzylethyleneamine; salts with inorganic acids such as hydrochloric acid, hydrobromic acid, sulfuric acid, and phosphoric acid; salts with organic carboxylic acids such as formic acid, acetic acid, trifluoroacetic acid, maleic acid, and tartaric acid; addition salts with sulfonic acids such as methanesulfonic acid, benzenesulfonic acid, and p-toluenesulfonic acid; and salts or addition salts with basic or acidic amino acids such as arginine, aspartic acid, and glutamic acid; can be mentioned.

Peptide [I] exhibits very excellent antibacterial action on *P. acnes,* and is effective in the prevention or treatment of *P. acnes* infection and a disease resulting from *P. acnes* infection, for example, acne, sarcoidosis, uveitis, conjunctivitis, keratitis, corneal phlycten, endocarditis, spondylarthritis and the like, preferably acne and sarcoidosis.

The above-described acne includes acne vulgaris, neonatal acne, acne conglobata, demodex acne, acneiform eruption, acne by exogenous chemical substance, necrotic acne, summer acne and the like, and is preferably acne vulgaris.

The above-described sarcoidosis includes sarcoidosis of the lungs, eyes, skin, lymph nodes, bones, joints, heart, nervous tissue, and other visceral organs, and is preferably ocular sarcoidosis.

Because acne is a disease resulting from *P. acnes* infection, and accompanied by hyperkeratosis and inflammation due to neutrophil induction, peptide [I], which exhibits antibacterial action on *P. acnes,* hyperkeratosis suppressive action, and suppressive action on inflammation due to neutrophil induction, is highly effective in the prevention or treatment of acne.

An antibacterial agent for *P. acnes* or prophylactic or therapeutic agent for a disease resulting from *P. acnes* infection according to the present invention is administered to mammals, including humans, in a dosage form known per se, for example, dosage forms such as external preparations such as ointments, creams, gels, gel creams, lotions, pastes, liniments, emulsions, solutions for external use, plasters, aerosols, inhalants, sprays, suppositories, enemas, dipping agents, patches, plasters, tapes, nasal drops, ear drops, eye drops, and ocular ointments; parenteral preparations such as injections (solutions, suspensions and the like), infusions, and intravenous drips; oral preparations such as tablets, granules, fine granules, capsules, microcapsules, pellets, fine powders, powders, pills, solutions for drinking, solutions, infusions, decoctions, extracts, suspensions (for example, olive oil), syrups, lemonades, elixirs, and troaches, and the like.

Although the dosage form of an antibacterial agent for *P. acnes* or prophylactic or therapeutic agent for a disease resulting from *P. acnes* infection according to the present invention can be selected as appropriate in consideration of the lesion site, the significance of the onset of efficacy and the like, it is preferable to administer the agent as

an external preparation. When the disease resulting from *P. acnes* infection is acne, dosage forms such as ointments, creams, gels, gel creams, lotions, pastes, liniments, emulsions, solutions for external use, plasters, sprays, dipping agents, patches, plasters, and tapes are particularly preferable out of external preparations. When the disease resulting from *P. acnes* infection is ocular sarcoidosis, dosage forms such as eye drops or ocular ointments are particularly preferable.

An antibacterial agent for *P. acnes* or prophylactic or therapeutic agent for a disease resulting from *P. acnes* infection according to the present invention is administered by a method suitable for each dosage form when used. For example, in the case of external preparations, this is sprayed, applied or spread directly to a required site such as skin or mucosa; in the case of tablets, pills, solutions for drinking, suspensions, emulsions, granules and capsules, this is administered orally; in the case of injections, this is administered intravenously, intramuscularly, intracutaneously, subcutaneously, intra-articularly or intraperitoneally; and in the case of suppositories, this is administered intrarectally.

These pharmaceutical compositions can be prepared as pharmaceutical preparations by a method known per se using various pharmaceutically acceptable organic or inorganic carriers conventional for pharmaceutical preparation, like bases, for example, cacao butter, white petrolatum, polyethylene glycol, propylene glycol, liquid paraffin, beeswax, olive oil, cacao oil, sesame oil, soy oil, camellia oil, peanut oil, beef tallow, lard, lanolin, methylparaben, polyoxyethylene hydrogenated castor oil, gelled hydrocarbon (for example, plastibase, poloid and the like), cetyl alcohol, stearyl alcohol and the like; excipients, for example, sucrose, starch, mannitol, sorbitol, lactose, glucose, cellulose, talc, calcium phosphate, calcium carbonate and the like; binders, for example, cellulose, methylcellulose, hydroxyethylcellulose, hydroxymethylcellulose, polypropylpyrrolidone, gelatin, gum arabic, polyethylene glycol, sucrose, starch, carboxymethylstarch, sodium alginate, dextrin, polyvinyl alcohol, stearic acid and the like; disintegrants, for example, starch, carboxymethylcellulose, hydroxypropylstarch, sodium hydrogen carbonate, calcium phosphate, calcium citrate and the like; lubricants, for example, magnesium stearate, aerosil, talc, sodium lauryl sulfate and the like; correctives, for example, citric acid, menthol, glycine, orange powder and the like; preservatives, for example, sodium benzoate, sodium bisulfite, methylparaben, propylparaben and the like; stabilizers, for example, citric acid, sodium citrate, acetic acid and the like; suspending agents, for example, methylcellulose, polyvinylpyrrolidone, aluminum stearate and the like; dispersing agents, for example, hydroxypropylmethylcellulose and the like; diluents, for example, purified water, physiological saline, ethanol, methanol, dimethylsulfoxide (DMSO) and the like; thickening agents, for example, gum tragacanth and the like, and the like.

Furthermore, as required, filler such as kaolin, bentonite, and zinc oxide; moisturizers such as glycerine and propylene glycol; supports; adhesives; auxiliaries; colorants; flavors; pH adjusters; extenders; solubilizers; buffer agents; humectants; surfactants; antioxidants; propellants; solvents; dissolution aids and the like may be used. Also, by selecting an appropriate solvent, peptide [I] can be used in the liquid preparation form as is.

When an antibacterial agent for *P. acnes* or prophylactic or therapeutic agent for a disease resulting from *P. acnes* infection according to the present invention is used as an external preparation for the skin, such as ointments, creams, gels, gel creams, lotions, pastes, liniments, emulsions, solutions for external use, plasters, sprays, patches, plasters or tapes, the skin absorption of pharmacologically effective ingredient can be facilitated to further enhance the therapeutic effect by further adding at least one kind of percutaneous absorption promoter selected from the group consisting of N-acylsarcosine or a salt thereof, a higher fatty acid ester that is a reaction product of a higher fatty acid having a carbon number of 10 to 18 and an alcohol having a carbon number of 1 to 20, a dicarboxylic acid having a carbon number of 2 to 10 or a salt thereof, a hydroxycarboxylic acid ester that is a reaction product of a hydroxycarboxylic acid having a carbon number of 3 to 6 and an alcohol having a carbon number of 1 to 20, and a fatty acid ethanolamide.

As examples of the above-described N-acylsarcosine, N-lauroylsarcosine, N-oleoylsarcosine, N-palmitoylsarcosine, coconut oil fatty acid sarcosine and the like can be mentioned; as examples of the salt thereof, the sodium salt, potassium salt, magnesium salt, calcium salt, aluminum salt and the like of the above-described N-acylsarcosine can be mentioned.

The above-described higher fatty acid ester is a reaction product of a higher fatty acid having a carbon number of 10 to 18 and an alcohol having a carbon number of 1 to 20. If the carbon number of the above-described higher fatty acid is too small, the product higher fatty acid ester becomes likely to volatilize; if the carbon number is too large, the percutaneous absorption effect decreases. Also, if the carbon number of the above-described alcohol is too large, the percutaneous absorption effect decreases.

As examples of the above-described higher fatty acid having a carbon number of 10 to 18, saturated aliphatic monocarboxylic acids such as capric acid, lauric acid, myristic acid, palmitic acid, and stearic acid; unsaturated aliphatic monocarboxylic acids such as palmitoleic acid, oleic acid, vaccenic acid, linolic acid, and linolenic acid; saturated aliphatic dicarboxylic acids such as sebacic acid, and the like can be mentioned.

As examples of the above-described alcohol having a carbon number of 1 to 20, aliphatic saturated alcohols such as methyl alcohol, ethyl alcohol, propyl alcohol, isopropyl alcohol, butyl alcohol, isobutyl alcohol, tertiary butyl alcohol, pentyl alcohol, hexyl alcohol, heptyl alcohol, octyl alcohol, capryl alcohol, nonyl alcohol, decyl alcohol, lauryl alcohol, myristyl alcohol, palmityl alcohol, and stearyl alcohol, and the like can be mentioned.

As examples of the above-described higher fatty acid ester, isopropyl myristate, isopropyl palmitate, isopropyl laurate, isopropyl stearate and the like can be mentioned.

The above-described dicarboxylic acid or a salt thereof has a carbon number of 2 to 10. If the carbon number is too small or too large, the percutaneous absorption effect decreases. As examples of the above-described dicarboxylic acid having a carbon number of 2 to 10, saturated aliphatic dicarboxylic acids such as oxalic acid, malonic acid, succinic acid, glutaric acid, adipic acid, pimelic acid, suberic acid and the like; unsaturated aliphatic dicarboxylic acids such as fumaric acid, maleic acid and the like; aromatic dicarboxylic acids such as phthalic acid, isophthalic acid, terephthalic acid and the like, and the like can be mentioned; as examples of the salt thereof, the sodium salt, potassium salt, magnesium salt, calcium salt, aluminum salt and the like of the above-described dicarboxylic acid can be mentioned.

The above-described hydroxycarboxylic acid ester is a reaction product of a hydroxycarboxylic acid having a carbon number of 3 to 6 and an alcohol having a carbon number of 1 to 20. If the carbon number of the above-described hydroxycarboxylic acid is too small, the product hydroxycarboxylic acid ester becomes likely to volatilize; if the carbon number is too large, the percutaneous absorption effect decreases. Also, if the carbon number of the above-described alcohol is too large, the percutaneous absorption effect decreases.

As examples of the above-described hydroxycarboxylic acid having a carbon number of 3 to 6, monocarboxylic acids such as lactic acid and glyceric acid; dicarboxylic acids such as malic acid and tartaric acid, and the like can be mentioned. As the above-described alcohol having a carbon number of 1 to 20, those used in the above-described reaction of higher fatty acid ester and the like can be mentioned. As examples of the above-described hydroxycarboxylic acid ester, myristyl lactate, cetyl lactate and the like can be mentioned.

As examples of the above-described fatty acid ethanolamide, fatty acid monoethanolamides, fatty acid diethanolamides; alkylene oxide adducts thereof, and the like can be mentioned. As examples of the above-described fatty acid ethanolamide, lauric monoethanolamide, lauric diethanolamide, lauroyl monoethanolamide, palmitic monoethanolamide, palmitic diethanolamide, myristic monoethanolamide, myristic diethanolamide, lauric/myristic monoethanolamide, coconut oil fatty acid monoethanolamide, coconut oil fatty acid diethanolamide, polyoxyethylene adduct lauroyl monoethanolamide, polyoxyethylene adduct coconut oil fatty acid monoethanolamide and the like can be mentioned.

As the above-described percutaneous absorption accelerator that can be contained in an antibacterial agent for *P. acnes* and prophylactic or therapeutic agent for a disease resulting from *P. acnes* infection according to the present invention, N-lauroylsarcosine, isopropyl myristate, isopropyl palmitate, fumaric acid, maleic acid, myristyl lactate, cetyl lactate, and lauric diethanolamide are particularly preferable. Although the above-described percutaneous absorption accelerator is preferably one of the above-described ones but is not limited thereto; conventionally known ones can be used.

The amount of peptide [I] contained in an antibacterial agent for *P. acnes* or prophylactic or therapeutic agent for a disease resulting from *P. acnes* infection according to the present invention is not subject to limitation, and is appropriately selected from over a broad range in consideration of dosage form, route of administration and the like; preferably, peptide [I] is contained in the range of $10^{-10}$ to 20% by weight, more preferably in the range of $10^{-7}$ to 10% by weight. In particular, when the agent is used as an external preparation, the amount of peptide [I] is in the range of 0.01 to 20% by weight, preferably in the range of 0.1 to 5% by weight.

The dose of an antibacterial agent for *P. acnes* or prophylactic or therapeutic agent for a disease resulting from *P. acnes* infection according to the present invention is not subject to limitation, is appropriately selected from over a broad range in consideration of patient body weight, age, dosage form, route of administration, symptoms, affected portion size and the like. The usual daily dose is in the range of about 10 pg/kg to 100 mg/kg, preferably in the range of about 0.1 mg/kg to 10 mg/kg based on peptide [I], and the agent is administered in one to four divided portions.

The present invention also relates to a cosmetic for the prevention or treatment of acne comprising peptide [I] or a pharmaceutically acceptable salt thereof as an active ingredient.

As examples of the cosmetic, skin cosmetics in the form of various compositions such as cosmetic lotions, creams, emulsions, and packs can be mentioned. When a composition comprising peptide [I] is prepared as a cosmetic, in addition to the above-described additives that can be added when preparing peptide [I] as a pharmaceutical composition, additives conventional in the cosmetic field such as cosmetic activators, antiseptics, flavoring agents, fillers, pigments, UV blockers, odor absorbents, dyes, and moisturizers (glycerol and the like), can also be added, as long as the advantageous characteristics of a composition according to the present invention are not adversely affected. For example, a cosmetic lotion can be produced by dissolving a moisturizer such as glycerine, a skin nutrient and the like in purified water, dissolving an antiseptic, a flavoring agent and the like in alcohol, blending the two solutions, solubilizing the mixture at room temperature, and adding peptide [I] or a pharmaceutically acceptable salt thereof to the alcohol moiety.

A cosmetic for the prevention or treatment of acne according to the present invention is normally used (administered)by spraying, applying or coating an effective amount directly to a target site such as skin.

The amount of peptide [I] contained in a cosmetic for the prevention or treatment of acne according to the present invention is appropriately selected from over a broad range according to the form of the cosmetic composition and the like, and is preferably in the range of 0.001 to 20% by weight, more preferably 0.01 to 5% by weight, based on

peptide [I].

The amount of use of a cosmetic for the prevention or treatment of acne according to the present invention is not subject to limitation, and is appropriately selected from over a broad range in consideration of the form of the composition, use site and the like. The cosmetic is usually used in the range of about 0.01 g to 10 g per use, 1 to 4 times per day.

The present invention is hereinafter described in more detail by means of the following examples, which, however, are not to be construed as limiting the present invention.

(Example 1)

Six strains of *P. acnes* isolated at an educational hospital in Japan and identified and stocked at the inventors' institution in 1991 were used as the test strains. Each test strain was subjected to anaerobic culture in GAM agar medium (manufactured by Nissui Pharmaceutical Co., Ltd.) at 35°C for 48 hours, and the developed colonies were inoculated to GAM broth (manufactured by Nissui Pharmaceutical Co., Ltd.) and subjected to anaerobic culture at 35°C for 18 to 20 hours. They were diluted 100 fold with a buffered physiological saline containing gelatin (BSG; a solution of 8.5 g of NaCl, 0.3 g of $KH_2PO_4$, 0.6 g of $Na_2HPO_4$, and 0.1 g of gelatin dissolved in 1000 mL of sterile water) and used as the inoculum liquid. Peptide [I] was dissolved in dimethylsulfoxide (DMSO) and sequentially diluted 2 fold with DMSO. This was dispensed to sterile dishes to obtain a final concentration of DMSO of 1%, and 9.9 mL of sterilized brain heart infusion agar (BBL) was added; agar plates of two-fold serial dilutions were prepared to obtain final concentrations of peptide [I] of 128 μg/mL to 0.0039 μg/mL. The inoculum liquid was inoculated to the agar plates by the stamp method and subjected to anaerobic culture at 35°C for 48 hours. MIC determinations were performed in accordance with NCCLS (M11-A4). The results are shown in Table 1.

Table 1

| Bacterial species | Strain number | MIC (μg/ml) |
|---|---|---|
| *Propionibacterium acnes* | #11053 | ≦0.0039 |
| | #11054 | ≦0.0039 |
| | #11055 | ≦0.0039 |
| | #11056 | ≦0.0039 |
| | #11057 | ≦0.0039 |
| | #11058 | ≦0.0039 |

(Results)

As is evident from Table 1, peptide [I] was shown to possess potent antibacterial activity on all the six strains of *P. acnes* used in the test.

(Example 2)

After normal human epidermal keratinocytes (purchased from Sanko Junyaku Co., Ltd.) were prepared to a cell density of $2\times10^4$ cells/mL using normal human epidermal keratinocyte growth medium (KGM2), the cells were sown to a 96-well plate at 100 μL per well ($2\times10^3$ cells/well). After cultivation at 37°C in a CO2 incubator for 2 to 3 hours, peptide [I] dissolved in 0.02% methanol-KGM2 was added at 100 μL/well. The final methanol concentration was 0.01%. After the cells were cultured for 3 days, the medium was replaced with a fresh one, and the cells were further cultured for 3 days. After the cultivation, a Cell counting kit reagent (manufactured by Wako Pure Chemical Industries, Ltd.) was added at 20 μL/well, and a color developing reaction was carried out in a CO2 incubator. Reaction time was set to 1 to 4 hours while monitoring the color developing state. After the reaction, absorbance at 450 nm was determined using a plate reader to be used as the index of cell growth. Experiments were performed in triplicate repeatedly three times, the absorbance was compared with that from the peptide [I] non-addition group, and the results were statistically analyzed by Dunnet's multiple comparison test. The results are shown in Table 2.

Table 2

| Peptide [I] concentration (M) | Absorbance (450 nm) |
|---|---|
| 0 | 1.067 |
| $1.0\times10^{-9}$ | 1.126 |
| $3.0\times10^{-9}$ | 1.088 |
| $1.0\times10^{-8}$ | 0.848 |
| $3.0\times10^{-8}$ | 0.123** |
| $1.0\times10^{-7}$ | 0.122** |
| **: P<0.01 (Dunnet's multiple comparison test) | |

(Results)

As is evident from Table 2, peptide [I] was shown to possess potent normal human epidermal keratinocyte growth suppressive activity.

(Example 3)

Twenty microliters of 50 μg/mL TPA dissolved in 1% DMSO-acetone solution was applied to each face of the right auricle of a female BDF1 mouse (7 weeks of age) (2 μg/auricle) to induce dermatitis. Peptide [I] was used in solution in acetone/methanol (1:1); 20 μL of this solution was applied to each face of the right auricle at 24 hours and 1 hour before TPA stimulation. The thickness of the right auricle was measured using a Peacock dial thickness gauge at 6 hours and 24 hours after the TPA stimulation to be used as the index of edema. Twenty-four hours after the TPA stimulation, the right auricle was collected, and MPO activity was determined to be used as the index of neutrophil infiltration. The measurements were performed on five animals in each group. The results were obtained as suppression rates (%) for each of auricle thickness and MPO activity using the equation below.

Suppression rate (%) = [(mean value for peptide [I] non-administration group - mean value for peptide [I] administration group)/(mean value for peptide [I] non-administration group - mean value for non-TPA-application group)] × 100

The results were statistically analyzed by Dunnet's multiple comparison test. The results are shown in Table 3.

Table 3

| Peptide [I] concentration (% by weight) | Suppression rate (%) | | |
|---|---|---|---|
| | Edema (6 hr) | Edema (24 hr) | MPO activity (24 hr) |
| 0.3 | 58.3** | 42.0* | 75.5** |
| 1.0 | 81.1** | 78.0** | 91.0** |
| 3.0 | 76.4** | 80.0** | 96.0** |
| *: P<0.05, **: P<0.01 (Dunnet's multiple comparison test) | | | |

(Results)

As is evident from Table 3, peptide [I] potently suppressed edema and neutrophil infiltration in TPA-induced

mouse dermatitis. From this result, it was suggested that the peptide [I] of the present invention might exhibit suppressive action on inflammation due to neutrophil infiltration.

(Example 4)

Ten microliters of 1 mg/mL calcium ionophore (A23187) dissolved in 10% DMSO-acetone solution was applied to each face of the right auricle of a female BALB/c mouse (5 weeks of age) (20 μg/auricle) to induce dermatitis. Peptide [I] was used in solution in acetone/methanol (1:1); 20 μL of this solution was applied to each face of the right auricle at 24 hours and 1 hour before A23187 stimulation. The thickness of the right auricle was measured using a Peacock dial thickness gauge at 6 hours and 24 hours after the A23187 stimulation to be used as the index of edema. At 24 hours after the A23187 stimulation, the right auricle was collected, and MPO activity was determined to be used as the index of neutrophil infiltration. The measurements were performed on five animals in each group. The results were obtained as suppression rates (%) for each of auricle thickness and MPO activity using the equation below.

Suppression rate (%) = [(mean value for peptide [I] non-administration group - mean value for peptide [I] administration group)/(mean value for peptide [I] non-administration group - mean value for non-A23187-application group)] × 100

The results were statistically analyzed by Dunnet's multiple comparison test. The results are shown in Table 4.

Table 4

| Peptide [I] concentration (% by weight) | Suppression rate (%) | | |
|---|---|---|---|
| | Edema (6 hr) | Edema (24 hr) | MPO activity (24 hr) |
| 0.3 | 63.6** | -6.9 | -3.2 |
| 1.0 | 86.4** | 29.4** | 41.6** |
| 3.0 | 77.2** | 51.9** | 69.6** |
| **: P<0.01 (Dunnet's multiple comparison test) | | | |

(Results)

As is evident from Table 4, peptide [I] potently suppressed edema and neutrophil infiltration in A23187-induced mouse dermatitis. From this result, it was suggested that peptide [I] might exhibit suppressive action on inflammation due to neutrophil infiltration.

(Example 5)

| | |
|---|---|
| Peptide [I] | 1 mg |
| Plastibase (manufactured by Taisho Pharmaceutical Co., Ltd.) | 1 g |

After 1 mg of peptide [I] is finely milled using a mortar, 1 g of plastibase is added, and they are thoroughly mixed on the mortar to prepare an ointment.

(Example 6)

| | |
|---|---|
| Peptide [I] | 0.2 mg |
| Methanol | 12.5 μL |
| Plastibase (manufactured by Taisho Pharmaceutical Co., Ltd.) | 1 g |

After 0.8 mg of peptide [I] is dissolved in 50 μL of methanol, 12.5 μL of the obtained methanol containing the peptide of the present invention is thoroughly mixed with 1 g of Plastibase on a mortar to prepare an ointment.

(Example 7)

| | |
|---|---|
| Peptide [I] | 0.5 mg |
| DMSO | 0.1 mL |
| 5% by weight aqueous solution of gum arabic | 1.9 mL |

After 0.1 ml of DMSO is added to 0.5 mg of peptide [I] to dissolve the peptide, 0.1 mL of the obtained DMSO solution containing the peptide of the present invention is added little by little to 1.9 mL of 5% by weight of gum arabic solution with stirring to prepare a homogeneously suspended liquid preparation.

(Example 8)

| | |
|---|---|
| Peptide [I] | 0.5 mg |
| Physiological saline | 2 mL |

A small amount of physiological saline is added to 0.5 mg of peptide [I] and the obtained mixture is subjected to ultrasonication to prepare a homogeneous suspension. Physiological saline is added to the suspension to prepare 2 mL of a liquid preparation.

(Example 9)

| | |
|---|---|
| Peptide [I] | 25 mg |
| Ethanol | 2.5 mL |
| Polyoxyethylene hydrogenated castor oil 60 | 1 g |
| Physiological saline | 96.5 mL |

After 25 mg of peptide [I] is dissolved in 2.5 mL of ethanol, 1 g of polyoxyethylene hydrogenated castor oil 60 is added thereto. The obtained solution is added little by little to 96.5 mL of physiological saline with stirring to prepare a liquid preparation.

(Example 10)

| | |
|---|---|
| Peptide [I] | 10 mg |
| Macrogol 400 | 50 mL |
| Physiological saline | q.s. |

After 10 mg of peptide [I] is dissolved in 50 mL of macrogol 400, physiological saline is added thereto to prepare 100 mL of a liquid preparation.

## Industrial Applicability

The present invention relates to an antibacterial agent for *P. acnes;* a prophylactic or therapeutic agent for a disease resulting from *P. acnes* infection, such as acne and sarcoidosis; a cosmetic for the prevention or treatment of acne, and the like, and is useful in the pharmaceutical industry.

This application is based on a patent application No. 2003-323063 filed in Japan, the contents of which are hereby incorporated by reference.

## Claims

1. An antibacterial agent for *Propionibacterium acnes* comprising, as an active ingredient, a peptide represented by the formula [I]:

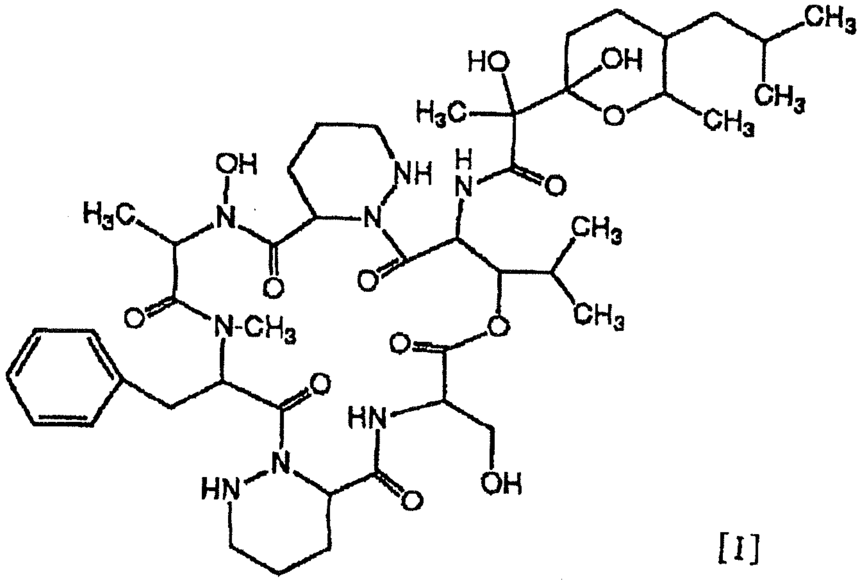

[I]

or a pharmaceutically acceptable salt thereof.

2. A prophylactic or therapeutic agent for a disease resulting from *Propionibacterium acnes* infection, which comprises as an active ingredient a peptide represented by the formula [I]

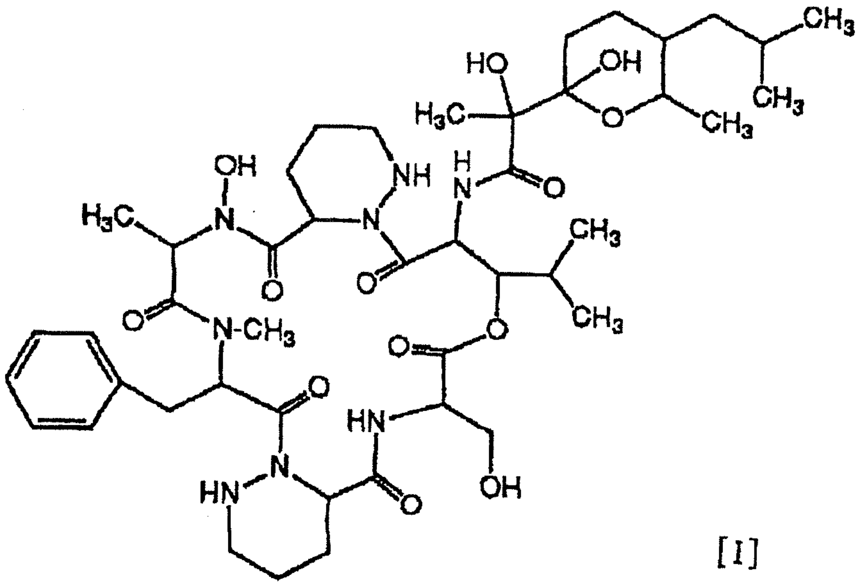

[I]

or pharmaceutically acceptable salt thereof.

3. The prophylactic or therapeutic agent of claim 2, wherein the disease resulting from *Propionibacterium acnes* infection is a disease selected from the group consisting of acne, sarcoidosis, uveitis, conjunctivitis, keratitis, corneal phlyctena, endocarditis and spondylarthritis.

4. The prophylactic or therapeutic agent of claim 3, wherein the disease resulting from *Propionibacterium acnes* infection is acne.

5. The prophylactic or therapeutic agent of claim 4, wherein the acne is acne vulgaris.

6. The prophylactic or therapeutic agent of claim 3, wherein the disease resulting from *Propionibacterium acnes* infection is sarcoidosis.

7. The prophylactic or therapeutic agent of claim 6, wherein the sarcoidosis is ocular sarcoidosis.

8. The agent of any one of claims 1 to 7, wherein the dosage form is an external preparation.

9. A cosmetic for the prevention or treatment of acne comprising a peptide represented by formula [I]

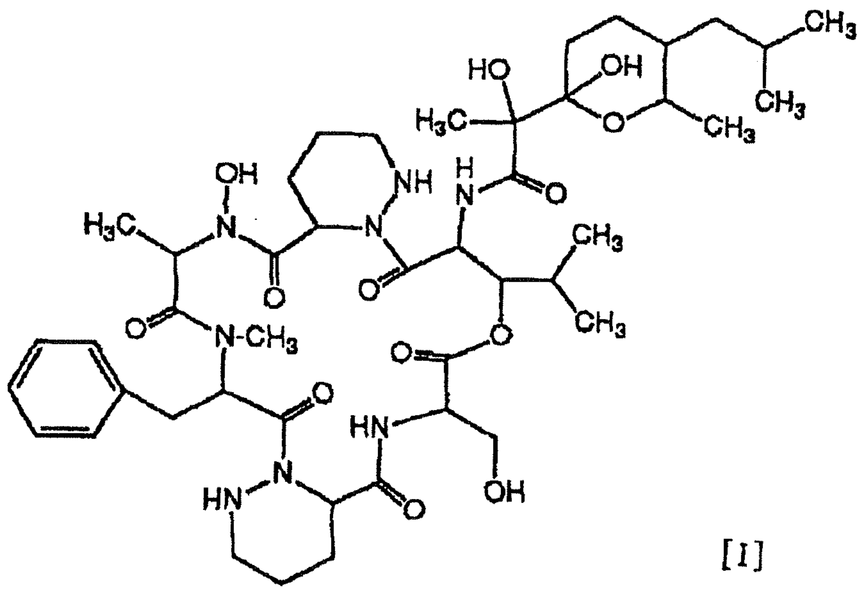

[I]

or a pharmaceutically acceptable salt thereof as an active ingredient.

**10.** A prophylactic or therapeutic method for *Propionibacterium acnes* infection comprising using a peptide represented by formula [I]

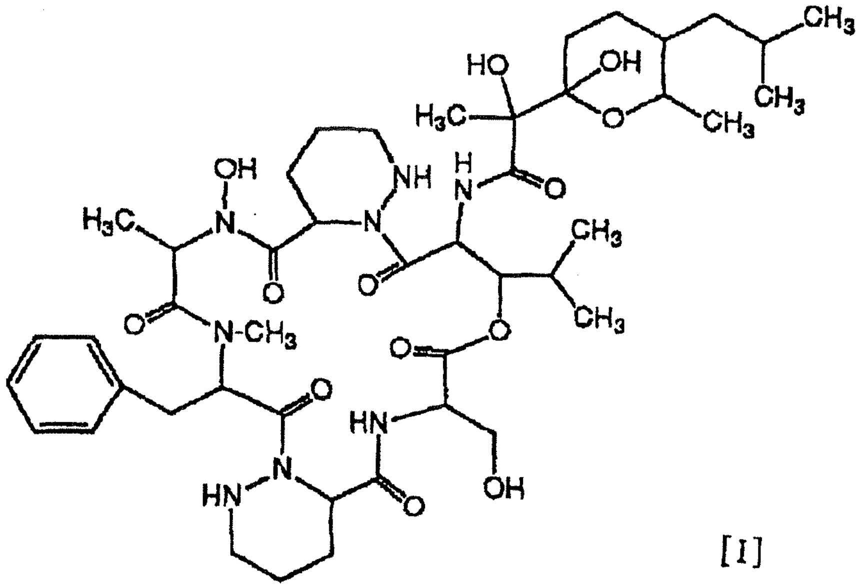

[I]

or a pharmaceutically acceptable salt thereof.

**11.** A prophylactic or therapeutic method for a disease resulting from *Propionibacterium acnes* infection comprising using a peptide represented by formula [I]

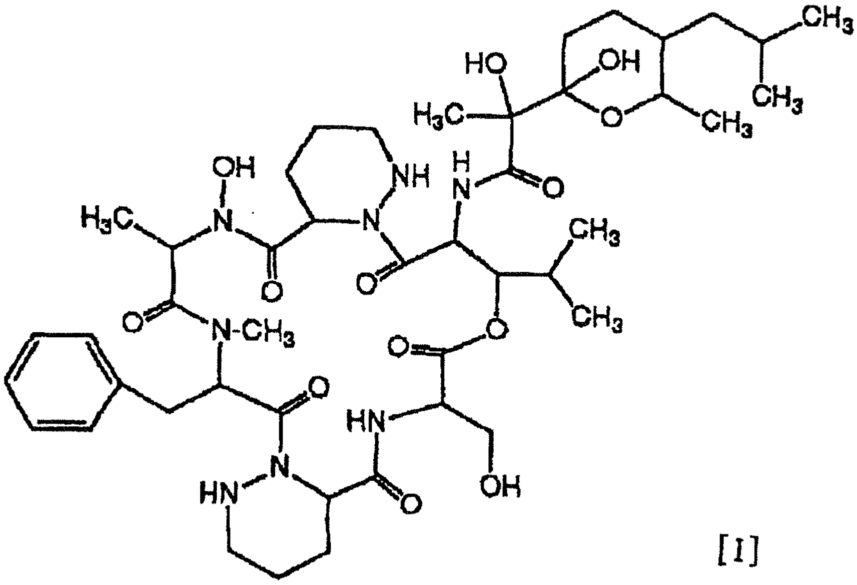

[I]

or a pharmaceutically acceptable salt thereof.

**12.** A prophylactic or therapeutic method for acne comprising using a cosmetic comprising a peptide represented by formula [I]

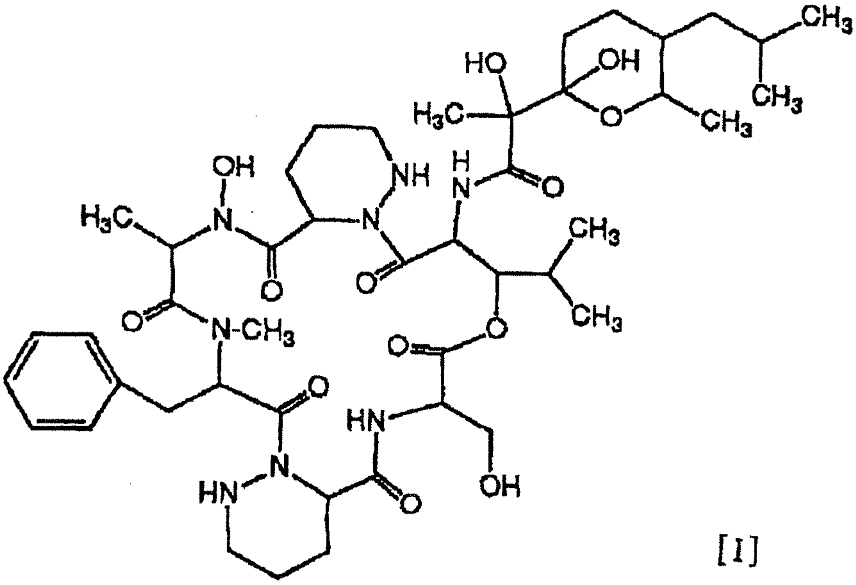

[I]

or a pharmaceutically acceptable salt thereof as an active ingredient.

**13.** A use of a peptide represented by formula [I]

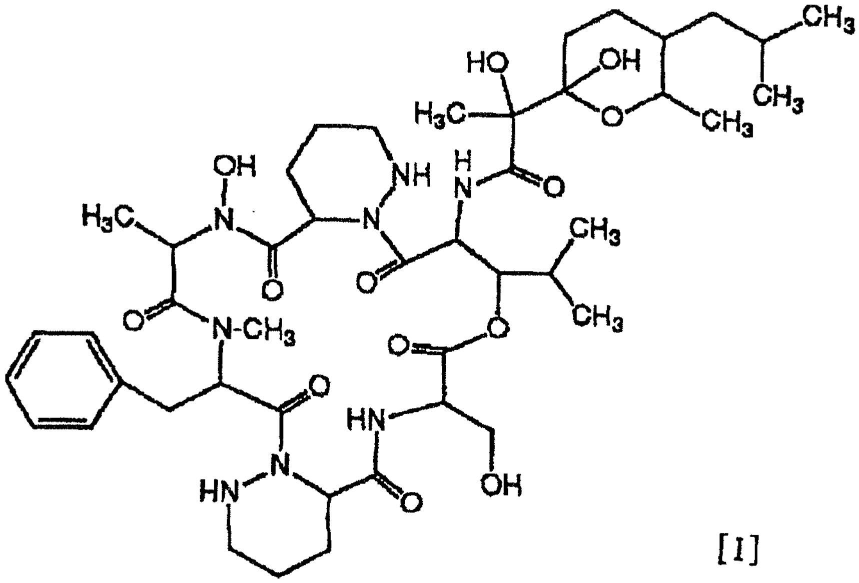

[I]

or a pharmaceutically acceptable salt thereof for producing an antibacterial agent for *Propionibacterium acnes*.

**14.** A use of a peptide represented by formula [I]

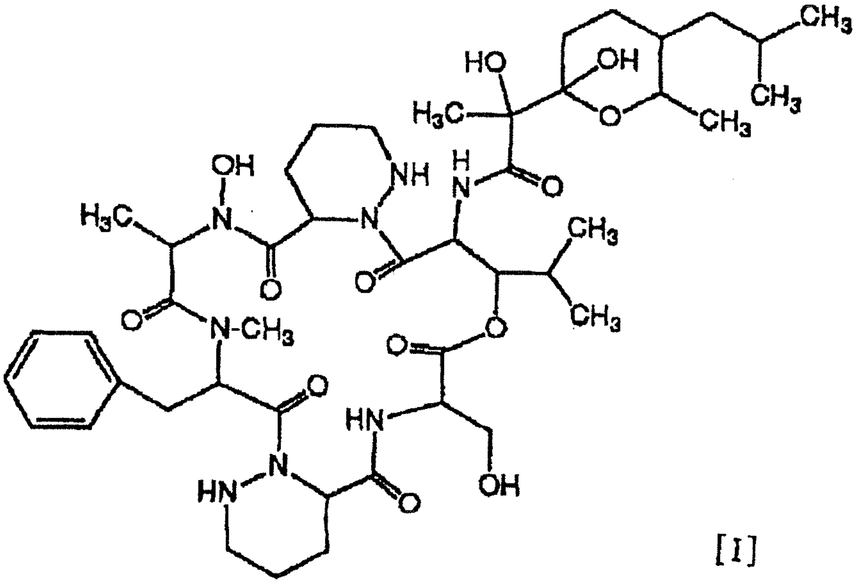

[I]

or a pharmaceutically acceptable salt thereof for producing a prophylactic or therapeutic agent for a disease resulting from *Propionibacterium acnes* infection.

**15.** A use of a peptide represented by formula [I]

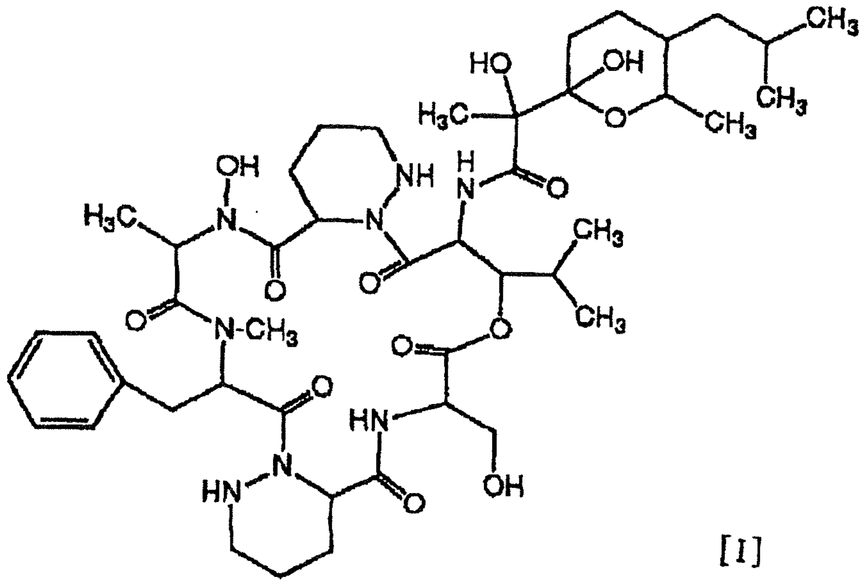

[I]

or a pharmaceutically acceptable salt thereof for producing a cosmetic for the prevention or treatment of acne.

**INTERNATIONAL SEARCH REPORT**

International application No.
PCT/JP2004/013861

A. CLASSIFICATION OF SUBJECT MATTER
Int.Cl$^7$ A61K38/12, 7/00, A61P31/04, 17/10, 27/02, 19/02, 35/00

According to International Patent Classification (IPC) or to both national classification and IPC

B. FIELDS SEARCHED

Minimum documentation searched (classification system followed by classification symbols)
Int.Cl$^7$ A61K38/12, 7/00, A61P31/04, 17/10, 27/02, 19/02, 35/00

Documentation searched other than minimum documentation to the extent that such documents are included in the fields searched

Electronic data base consulted during the international search (name of data base and, where practicable, search terms used)
CAPLUS(STN)

C. DOCUMENTS CONSIDERED TO BE RELEVANT

| Category* | Citation of document, with indication, where appropriate, of the relevant passages | Relevant to claim No. |
|---|---|---|
| A | WO 96/12732 A (Sekisui Chemical Co., Ltd.), 02 May, 1996 (02.05.96), & EP 792886 A1 & US 5858971 A & JP 8-119993 A & JP 8-119992 A & JP 8-176189 A & JP 231590 A & JP 9-176188 A | 1-9,13-15 |
| A | JP 10-259141 A (Sekisui Chemical Co., Ltd.), 29 September, 1998 (29.09.98), (Family: none) | 1-9,13-15 |
| A | JP 11-080020 A (Sekisui Chemical Co., Ltd.), 23 March, 1999 (23.03.99), (Family: none) | 1-9,13-15 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 December, 2004 (17.12.04) | 11 January, 2005 (11.01.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/013861

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-338644 A (Sekisui Chemical Co., Ltd.), 22 December, 1998 (22.12.98), (Family: none) | 1-9,13-15 |
| A | JP 10-067677 A (Sekisui Chemical Co., Ltd.), 10 March, 1998 (10.03.98), (Family: none) | 1-9,13-15 |
| A | JP 10-120590 A (Sekisui Chemical Co., Ltd.), 12 May, 1998 (12.05.98), (Family: none) | 1-9,13-15 |
| A | JP 2002-322018 A (LG Household and Health Care Ltd.), 08 November, 2002 (08.11.02), & US 2003/031690 A1 & KR 2002-075632 A | 1-9,13-15 |
| A | Yoshinobu ESEKI, "Naiinsei Kansensho to Shiteno Sarcoidosis", Saishin Igaku, 2001, Vol.56, No.12, pages 2756 to 2764 | 1-9,13-15 |

Form PCT/ISA/210 (continuation of second sheet) (January 2004)

**INTERNATIONAL SEARCH REPORT**

International application No.
PCT/JP2004/013861

**Box No. II Observations where certain claims were found unsearchable (Continuation of item 2 of first sheet)**

This international search report has not been established in respect of certain claims under Article 17(2)(a) for the following reasons:

1. [X] Claims Nos.: 10-12
because they relate to subject matter not required to be searched by this Authority, namely:
Claims 10 to 12 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)